(19) Europäisches Patentamt
European Patent Office
Office européen des brevets

(11) **EP 2 354 117 B1**

(12) **EUROPÄISCHE PATENTSCHRIFT**

(45) Veröffentlichungstag und Bekanntmachung des Hinweises auf die Patenterteilung:
**24.09.2014 Patentblatt 2014/39**

(51) Int Cl.:
***C07C 205/06*** *(2006.01)*   ***C07C 201/08*** *(2006.01)*

(21) Anmeldenummer: **11152982.2**

(22) Anmeldetag: **02.02.2011**

(54) **Verfahren zur kontinuierlichen Herstellung von Nitrobenzol**

Method for continuous production of Nitrobenzol

Procédé destiné à la fabrication continue de nitrobenzène

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.02.2010 DE 102010006984**

(43) Veröffentlichungstag der Anmeldung:
**10.08.2011 Patentblatt 2011/32**

(73) Patentinhaber: **Bayer Intellectual Property GmbH 40789 Monheim (DE)**

(72) Erfinder:
• **Münnig, Jürgen 41564 Kaarst (DE)**
• **Pennemann, Bernd 51467 Bergisch Gladbach (DE)**
• **Rausch, Andreas 41464 Neuss (DE)**

(74) Vertreter: **BIP Patents c/o Bayer Intellectual Property GmbH Creative Campus Monheim Alfred-Nobel-Straße 10 40789 Monheim (DE)**

(56) Entgegenhaltungen:
**EP-A1- 1 816 117    EP-A1- 2 070 907 EP-A2- 0 373 966    EP-A2- 0 708 076 EP-A2- 1 291 078**

**Beschreibung**

Die Erfindung betrifft ein Verfahren zur kontinuierlichen Herstellung von Nitrobenzol durch Nitrierung von Benzol mit Mischsäure, bei dem der Druck vor dem Nitrierreaktor um 15 bar bis 25 bar über dem Druck in der Gasphase des Phasentrennapparates zur Trennung von Rohnitrobenzol und Absäure liegt.

Die vorliegende Erfindung betrifft ein kontinuierliches Verfahren zur Herstellung von Nitrobenzol, das im Wesentlichen dem Konzept der adiabaten Nitrierung von Benzol durch ein Gemisch von Schwefel- und Salpetersäure (sog. *Mischsäure*) entspricht. Ein solches Verfahren wurde erstmals in US-A-2 256 999 beansprucht und ist in heutigen Ausführungsformen beispielsweise in EP 0 436 443 B1, EP 0 771 783 B1 und US 6 562 247 B2 beschrieben. Die Verfahren mit adiabater Reaktionsführung zeichnen sich insbesondere dadurch aus, dass keine technischen Maßnahmen ergriffen werden, um der Reaktionsmischung Wärme zu- oder abzuführen.

Den beschriebenen adiabaten Verfahren ist gemein, dass die Ausgangsstoffe Benzol und Salpetersäure in einem großen Überschuss an Schwefelsäure zur Reaktion gebracht werden. Die Schwefelsäure nimmt dann die frei werdende Reaktionswärme und das bei der Reaktion gebildete Wasser auf.

Zur Durchführung der Reaktion werden im Allgemeinen Salpetersäure und Schwefelsäure zur so genannten Mischsäure (auch Nitriersäure genannt) vermischt, und Benzol wird in diese Mischsäure hinein dosiert. Das so erhaltene Verfahrensprodukt reagiert mit der Salpetersäure oder mit in der Mischsäure gebildeten "Nitroniumionen" im Wesentlichen zu Wasser und Nitrobenzol. Benzol wird in mindestens - bezogen auf die molare Salpetersäuremenge - stöchiometrischer Menge eingesetzt, bevorzugt aber im 2%igem bis 10%igem Überschuss im Vergleich zur stöchiometrisch benötigten Benzolmenge.

Wichtigstes Kriterium zur Beschreibung der Güte eines adiabaten Verfahrens zur Nitrierung aromatischer Kohlenwasserstoffe ist der Gehalt des Produktes an unerwünschten Nebenprodukten der Reaktion, die durch mehrfache Nitrierung oder Oxidation des aromatischen Kohlenwasserstoffes oder des Nitroaromaten gebildet werden. Im Falle der Nitrierung von Benzol wird dabei stets der Gehalt an Dinitrobenzol und an Nitrophenolen, insbesondere des als brisant einzustufenden Trinitrophenols (Pikrinsäure) diskutiert.

Um Nitrobenzol mit besonders hohen Selektivitäten zu erhalten, wurde die Art der zu verwendenden Mischsäure detailliert festgelegt (EP 0 373 966 B1, EP 0 436 443 B1 und EP 0 771 783 B1), sowie darauf hingewiesen, dass der Gehalt an Nebenprodukten durch die Höhe der Maximaltemperatur bestimmt wird (EP 0 436 443 B1, Spalte 15, Z. 22 - 25). Auch ist bekannt, dass ein hoher Anfangsumsatz vorteilhaft ist für eine hohe Selektivität und dass diese dann erreicht wird, wenn zu Beginn der Reaktion eine optimale Durchmischung herbeigeführt wird (EP 0 771 783 B1, Absatz [0014]).

Der kostengünstigen und effizienten Gestaltung der erstmaligen Mischung (Dispergierung) und der wiederholten Mischung (Redispergierung) von Aromaten in die Mischsäure widmen sich zahlreiche Untersuchungen, die als Ergebnis für die Durchmischung sowohl Düsen (EP 0 373 966 B1, EP 0 771 783 B1) als auch speziell geformte statische Dispergierelemente vorschlagen (EP 0 489 211 B1, EP 0 779 270 B1, EP 1 291 078 A1, und US 6 562 247 B2), wobei beide Konzepte auch kombiniert werden können.

Kommen für die Durchmischung statische Mischelemente (Dispergierelemente) zum Einsatz, so ist der Druckverlust an diesen statischen Mischelementen entscheidend für die Güte der Durchmischung. Der Druck vor dem Reaktor muss mindestens so groß sein wie die Summe aus den Druckverlusten aller Dispergierelemente in dem Reaktor; ggf. müssen auch noch weitere Faktoren berücksichtigt werden, wie der statische Druck der Flüssigkeitssäule im Reaktor und der Druck im Phasentrennapparat. Unter "Druck vor dem Reaktor" wird im Rahmen dieser Erfindung der absolute Druck verstanden, unter dem die flüssigen Edukte unmittelbar vor und daher auch bei Eintritt in den Reaktor stehen.

Gemäß der Lehre des Standes der Technik ist der gesamte Druckverlust über den Reaktor und ggf. weiteren dem Reaktor nachgeschalteten Apparaten (und damit auch der Druck vor dem Reaktor) *möglichst gering zu halten;* siehe bspw. EP 1 291 078 A2, Abschnitt [0017]. Ein anderes Beispiel wird in EP 2 070 907 A1 beschrieben, wo es heißt, dass ein Anstieg des absoluten Druckes vor dem Reaktor von 13,5 bar auf 14,5 bar infolge Ablagerungen von Metallsulfaten in den Dispergierelementen zu einer Verringerung des Durchsatzes an Schwefelsäure um 18 % führt (Beispiel 1). Der Stand der Technik lehrt also, dass hohe Druckverluste und damit hohe Absolutdrücke vor dem Reaktor zu vermeiden sind.

Ein Beispiel für die Vermischung von Aromat und Mischsäure mittels einer geeigneten Düse ohne statische Dispergierelemente ist EP 0 373 966 B1 zu entnehmen. Hier wird als geeigneter Bereich für den Arbeitsdruck ("back pressure" = Gegendruck in der Düse, also der Druck des flüssigen Eduktstromes (Aromat oder Mischsäure) bei Eintritt in den Reaktor, was gleichbedeutend ist mit dem Druck des betreffenden Eduktstromes vor dem Reaktor) ein Bereich zwischen 0,689 bar und 13,79 bar angegeben (S. 5, Z. 12 bis 13). Außerdem lehrt diese Schrift, dass es unter normalen Bedingungen nicht zu erwarten sei, einen höheren Druck als 11,03 bar zu benötigen (S. 5, Z. 15 bis 16).

Die mögliche Untergrenze für den Druck vor dem Reaktor ist zusätzlich dadurch festgelegt, dass das Benzol bei den gegebenen Bedingungen am Reaktoreingang flüssig vorliegen sollte (US 4 091 042, Spalte 2, Zeile 14 bis 17). Bezüglich der möglichen Obergrenze ist festzustellen, dass gemäß dem Stand der Technik der Druckverlust pro statischem Dispergierelement möglichst gering gehalten wird, da zur Überwindung eines höheren Druckverlustes beispielsweise eine Pumpe höherer Leistung benötigt wird, die wiederum zu höheren Kosten des Gesamtverfahrens führt (EP 1 291 078 A2, Abschnitt [0017]). Auch wird im Allgemeinen versucht, die Zahl und Dicke (Stabilität) der Dispergierelemente bevorzugt so niedrig wie möglich zu halten um damit die Kosten für die Dispergierelemente, für deren Herstellung oft nur der Sonderwerkstoff Tantal in Frage kommt, niedrig zu halten (EP 1 291 078 A1, Abschnitt [0018]).

Auch wird der Druck innerhalb des Reaktors durch das verwendete Material für den Rohrreaktor beschränkt. Unter den allgemein üblichen Bedingungen der adiabaten Nitrierung von Benzol, also bei 80 °C bis 150 °C und Verwendung von Schwefelsäure einer Konzentration zwischen 65 Massen-% und 80 Massen-%, sind lediglich Tantal, Teflon und Glas dauerhaft beständig. Hochlegierte Stähle können ebenfalls eingesetzt werden, insbesondere dann wenn die Schwefelsäure immer einen Restanteil an Salpetersäure enthält, da Salpetersäure auf den hochlegierten Stahl passivierend wirkt. Großtechnisch werden für die adiabate Nitrierung von Benzol vor allem mit Glas emaillierte Stahlrohre eingesetzt. Stahl-Emaille-Rohrsegmente sind gemäß DIN-Norm 2873 vom Juni 2002 für die Nenndruckstufe PN10 und höchstens für die Nenndruckstufe PN25 anzufertigen, wobei die Nenndruckstufe PN25 nur bei Rohrdurchmessern bis zu einer Nennweite von DN150 zulässig ist (Nenndruckstufen nach EN1333, Nennweite nach DIN EN ISO 6708). Wie dem Fachmann geläufig ist, ist der zulässige Betriebsdruck nicht identisch mit der Nenndruckstufe, sondern muss in Abhängigkeit von Temperatur und Werkstoff errechnet werden. Bei höheren Temperaturen wird der zulässige Betriebsdruck bedingt durch die Abnahme der zulässigen Werkstoffkennwerte entsprechend geringer. Beim Bau von chemischen Anlagen sind zusätzlich zu Apparaten und Rohrleitungen auch Armaturen (Ventile, Schieber, etc.) erforderlich, für die wiederum eigene Normen zu beachten sind. Aus diesen hohen Anforderungen ergibt sich, dass der mit dem Aufbau von großtechnischen Nitrieranlagen betraute Fachmann bemüht ist, den Druck innerhalb der Anlage, also insbesondere auch den Druck vor dem Reaktor, gering zu halten, solange ihm nicht bekannt ist, dass sich dadurch ein signifikanter Vorteil ergibt.

Den beschriebenen Verfahren des Standes der Technik gelingt es zwar, ein Rohnitrobenzol herzustellen, das einen geringen Gehalt an Nebenprodukten aufweist, also nur zwischen 100 ppm-300 ppm an Dinitrobenzol und 1500 ppm - 2500 ppm an Nitrophenolen enthält, wobei Pikrinsäure einen Anteil von 10 Massen-% - 50 Massen-% an den Nitrophenolen annehmen kann. Jedoch ist neben der Reinheit des Rohnitrobenzols von entscheidender Bedeutung für die industrielle Produktion, dass - insbesondere vor dem Hintergrund des ständig steigenden Bedarfes an Nitroaromaten, vor allem zur Herstellung von aromatischen Aminen und aromatischen Isocyanaten - die Herstellung der Nitroaromaten in möglichst kompakten Reaktionseinrichtungen durchgeführt werden kann. Eine wichtige Kenngröße zur Beschreibung des Verhältnisses zwischen produzierbarer Menge und Größe der Reaktionseinrichtung ist die *Raum-Zeit-Ausbeute* (RZA). Sie berechnet sich als Quotient aus der produzierbaren Menge der Zielverbindung pro Zeiteinheit und dem Volumen der Reaktionseinrichtung.

$$\text{RZA}\ [t_{Nitrobenzol} / (m^3_{Reaktionsraum} \cdot h)] = \text{produzierte Menge}\ [t_{Nitrobenzol} / h] / \text{Reaktionsraum}\ [m^3]$$

Im vorliegenden Falle der Nitrierung von Benzol berechnet sich die Raum-Zeit-Ausbeute zweckmäßiger Weise als Quotient aus der Produktion von Nitrobenzol in metrischen Tonnen pro Stunde und dem Volumen des Reaktionsraumes, wobei der *Reaktionsraum* als der Raum definiert wird, der mit der ersten Dispergierung von Benzol und Mischsäure beginnt und innerhalb dessen die Reaktion bis zu einem Umsatzgrad an Salpetersäure von mindestens 99 % abgeschlossen ist. Der Reaktionsraum ist dabei in einer technischen Vorrichtung zur Durchführung chemischer Reaktionen angeordnet, dem *Reaktor.* Im einfachsten Fall ist der Reaktionsraum identisch mit dem Innenvolumen des Reaktors. Mit der *ersten Dispergierung* ist in diesem Zusammenhang die erste intensive Vermischung von Benzol und Mischsäure gemeint. Diese findet, wie oben erläutert, entweder in einer Mischdüse oder in einem statischen Mischelement statt. Ein bloßes Zusammenführen eines Benzol- und eines Mischsäurestromes in einer gemeinsamen Zuleitung zum Reaktor hin ohne besondere Maßnahmen zur intensiven Vermischung der beiden Ströme gilt noch nicht als erste Dispergierung im Sinne dieser Erfindung.

Die Verweilzeit der Reaktionsmischung, bestehend aus aromatischer Verbindung und Mischsäure, innerhalb des Reaktionsraumes ist die **Reaktionszeit**.

Eine hohe Raum-Zeit-Ausbeute ist für die industrielle Anwendung eines Verfahrens von Vorteil, da dadurch kompakte Reaktionseinrichtungen gebaut werden können, die sich durch ein geringes Investitionsvolumen pro Kapazität auszeichnen. Hinsichtlich der Raum-Zeit-Ausbeute der Aromatennitrierung besteht gegenüber dem Stand der Technik noch deutlicher Verbesserungsbedarf.

Nun führen jedoch hohe Raum-Zeit-Ausbeuten bei adiabater Reaktionsführung einer Nitrierung (insbesondere bei konstanter Verweilzeit im Reaktor) unweigerlich zu hohen Temperaturdifferenzen (adiabaten Temperatursprüngen) zwischen Starttemperatur (der Temperatur der vermischten Edukte vor Einsetzen der Reaktion, bestimmt durch Berechnung der Mischtemperatur der einzelnen Ströme) und Reaktionsendtemperatur (der Temperatur nach im Wesentlichen vollständigem Salpetersäureumsatz); und wie dem Stand der Technik zu entnehmen ist, führen hohe Reaktionsendtemperaturen zu einer Verschlechterung der Selektivität (siehe z. B. EP 0 436 443 B1, Spalte 15, Z. 22 - 25).

Eine Aufgabe der vorliegenden Erfindung war es daher, ein Verfahren zur adiabaten Nitrierung von Aromaten bereitzustellen, bei dem eine hohe Raum-Zeit-Ausbeute erzielt wird, ohne dass die Produktqualität darunter leidet; d. h. Nitrobenzol wird in kompakten Reaktionseinrichtungen mit hoher Selektivität in hervorragenden Ausbeuten erhalten.

Die Aufgabe wurde gelöst durch ein Verfahren zur Herstellung von Nitrobenzol durch adiabate Nitrierung von Benzol mit Mischsäure enthaltend Schwefelsäure und Salpetersäure, wobei

(i) Benzol und Mischsäure entweder

a) getrennt voneinander
oder

b) gemeinsam, nachdem beide miteinander kontaktiert wurden

in einen Reaktor eingeleitet werden, wobei im Fall a) mindestens einer der Reaktanden Benzol oder Mischsäure, im Fall b) das nach Kontaktierung von Benzol und Mischsäure erhaltene Verfahrensprodukt bei Eintritt in den Reaktor unter einem Druck p1 steht;

(ii) Benzol und Mischsäure im Reaktor in 1 bis 30, bevorzugt 2 bis 20, besonders bevorzugt 6 bis 15 hintereinander angeordneten Dispergierelementen ineinander dispergiert werden;

(iii) das Reaktionsprodukt nach Verlassen des Reaktors einer Phasentrennung in einem Phasentrennapparat, in dessen Gasphase der Druck p2 herrscht, unterworfen wird;
wobei

(iv) die Druckdifferenz p1 - p2 15 bar bis 25 bar, bevorzugt 20 bar bis 25 bar beträgt.

Durch diese Vorgehensweise wird die Herstellung großer Mengen an Nitrobenzol in kompakten Reaktoren mit hervorragenden Ausbeuten und Selektivitäten ermöglicht.

Die Reaktanden Benzol und Mischsäure werden entweder a) getrennt voneinander über verschiedene Zuleitungen in den Reaktor eingeleitet, oder - bevorzugt - b) nachdem sie miteinander kontaktiert wurden, d. h. über eine gemeinsame Zuleitung. In der Ausführungsform b) findet bei diesem ersten Kontakt von Benzol und Mischsäure in einer gemeinsamen Zuleitung noch keine *intensive* Vermischung der beiden Ströme statt.

Der Druck p1 bei Eintritt in den Reaktor wird bevorzugt in einer der jeweiligen Zuleitungen (Benzol oder Mischsäure oder gemeinsame Zuleitung für beide) zum Reaktor hin gemessen.

Erfindungsgemäße Reaktoren sind bevorzugt Rührkessel-, Schlaufen oder Rohrreaktoren. Diese können in Reihe oder parallel verschaltet werden. Auch Kombinationen verschiedener Reaktortypen sind denkbar. Rohrreaktoren können zylindrisch oder konisch aufgebaut sein.

Erfindungsgemäße Dispergierelemente sind bevorzugt Siebböden und Lochbleche.

Im Anschluss an den Nitrierreaktor wird bevorzugt das rohe, flüssige Reaktionsprodukt einem Phasentrennapparat zugeführt, in dem sich zwei flüssige Phasen bilden, wobei die eine als Rohnitrobenzol (Nitrobenzol und Verunreinigungen) bezeichnet wird und die andere als Absäure (im Wesentlichen Wasser und Schwefelsäure). Rohnitrobenzol und Absäure werden, wie weiter unten noch genauer beschrieben, aufgearbeitet. Gleichzeitig mit der Bildung der beiden flüssigen Phasen entweichen in dem Phasentrennapparat Gase aus der flüssigen Phase, sodass der Phasentrennapparat auch eine dritte, gasförmige Phase aufweist.

Die Gasphase des Phasentrennapparates enthält im Wesentlichen Stickoxide sowie Wasser- und Benzoldampf. Diese Gase werden im Allgemeinen einem Abgassystem zugeführt. Der Druck p2 wird in dieser Gasphase gemessen.

Im Folgenden wird die Erfindung detailliert erläutert.

Erfindungsgemäß wird Benzol mit *Mischsäure* nitriert. Die eingesetzte Mischsäure enthält bevorzugt zwischen 64 Massen-% und 71 Massen-% Schwefelsäure und zwischen 2 Massen-% und 8 Massen-% Salpetersäure; besonders bevorzugt zwischen 66 Massen-% und 69 Massen-% Schwefelsäure und zwischen 3 Massen-% und 5 Massen-% Salpetersäure, wobei der Rest zu 100 Massen-% bevorzugt jeweils Wasser darstellt und sich die Massenprozentangaben auf die Gesamtmasse der Mischsäure beziehen. Die Konzentration der eingesetzten Schwefelsäure liegt bevorzugt zwischen 65 Massen-% und 80 Massen-%, die der Salpetersäure bevorzugt zwischen 62 Massen-% und 70 Massen-%; jeweils bezogen auf die Gesamtmasse der jeweiligen Säure.

Im erfindungsgemäßen Verfahren können Benzol und Mischsäure getrennt voneinander in den Nitrierreaktor eingeführt werden. Bevorzugt ist es jedoch, Benzol vorher in die Mischsäure einzudosieren und beide Reaktanden gemeinsam in den Reaktor zu leiten. Das Mengenverhältnis von Mischsäurestrom (in pro Stunde zugegebener Masse an Mischsäure) zu Benzolstrom (in pro Stunde zugegebener Masse an Benzol) wird auch als Phasenverhältnis bezeichnet und liegt bevorzugt zwischen 12 : 1 und 30 : 1, besonders bevorzugt zwischen 12 : 1 und 18 : 1.

Bei gemeinsamer Eindosierung von Benzol und Mischsäure in den Reaktor wird der Druck p1 bevorzugt in der gemeinsamen Zuleitung zum Reaktor hin gemessen, und zwar bevorzugt an einer Stelle unmittelbar vor dem Reaktor.

Alternativ kann man auch einen Druck p1a in der Zuleitung für die Mischsäure messen, bevor der Benzolstrom mit dem Mischsäurestrom vereinigt wird. Der dortige Druck p1a ist identisch mit dem Druck p1 bei Eintritt in den Reaktor, sofern sich durch die Art der Eindosierung von Benzol in den Mischsäurestrom kein Druckverlust für den Mischsäurestrom ergibt. Dies ist beispielsweise dann der Fall, wenn Benzol mit einer solchen Lanze oder einer Mischdüse in den Mischsäurestrom eingebracht wird, die bevorzugt nur einen geringen Teil (bevorzugt kleiner 10 %) der Querschnittsfläche der Mischsäureleitung einnimmt. Der Druck in der Benzolzuleitung (p1b) vor der Lanze oder Mischdüse ist bevorzugt größer als der Druck p1a in der Mischsäurezuleitung, besonders bevorzugt um 0,5 bar bis 10 bar größer.

Ergibt sich durch die Art der Eindosierung von Benzol in den Mischsäurestrom ein signifikanter Druckverlust im Mischsäurestrom, so ist es bevorzugt, den Druck p1 in der gemeinsamen Zuleitung zu messen.

Bei getrennter Zufuhr von Benzol und Mischsäure in den Reaktor ist es im Sinne dieser Erfindung bevorzugt, den Druck p1 in der Zuleitung für die Mischsäure zu messen, und zwar bevorzugt unmittelbar vor dem Reaktor, weil dies, infolge des durch die adiabate Fahrweise bedingten hohen Phasenverhältnisses, aussagekräftiger ist als es eine Messung in der Zuleitung für den Benzolstrom wäre.

Unabhängig von der genauen Ausführung der Zufuhr von Benzol und Mischsäure (getrennt oder gemeinsam, mit oder ohne Mischdüse) ist die Lage der für die Ermittlung des Druckes p1 relevanten Messstelle so zu wählen, dass der Druck, unter dem der relevante Stoffstrom (also beispielsweise das durch Kontaktierung von Benzol und Mischsäure erhaltene Verfahrensprodukt oder - bei getrennter Zufuhr - die Mischsäure) *bei Eintritt in den Reaktor* steht, korrekt ermittelt werden kann, dass also zwischen der Messstelle und der Stelle, an welcher der relevante Stoffstrom in den Reaktor eintritt, entweder kein signifikanter Druckverlust besteht oder der Druckverlust bekannt ist und rechnerisch berücksichtigt werden kann.

Die Druckmessung erfolgt im erfindungsgemäßen Verfahren mit dem Fachmann bekannten Vorrichtungen, bevorzugt mit digitalen Druckmessumformern mit einem Membranmanometer als Messgrößen-Aufnehmer.

Im erfindungsgemäßen Verfahren beträgt die Druckdifferenz zwischen p1 und dem Druck in der Gasphase des Phasentrennapparates, p2, 15 bar bis 25 bar, bevorzugt 20 bar bis 25 bar und ist damit höher als im Stand der Technik bislang üblich.

Erfindungsgemäß erfolgt die Nitrierung im Reaktor unter adiabaten Bedingungen, d. h. es werden keine technischen Maßnahmen ergriffen, um der Reaktionsmischung Wärme zu- oder abzuführen. Ein wichtiges Merkmal der adiabaten Nitrierung von aromatischen Kohlenwasserstoffen ist, dass die Temperatur der Reaktionsmischung proportional zum Reaktionsfortschritt, also proportional zum Salpetersäureumsatz, ansteigt. Dadurch wird eine Temperaturdifferenz zwischen der Temperatur der vermischten Reaktanden Aromat und Mischsäure vor Einsetzen der Reaktion (welche durch dem Fachmann bekannte thermodynamische Berechnungen ermittelt und folgend als **Starttemperatur** bezeichnet wird) und der Temperatur der Reaktionsmischung nach mindestens 99 % Salpetersäureumsatz (folgend als **Reaktionsendtemperatur** bezeichnet) erhalten. Dem Fachmann ist bekannt, dass die hier als Starttemperatur bezeichnete Größe im Allgemeinen vorteilhaft als Mischtemperatur der Mischsäure- und Benzolströme errechnet wird und die hier als Reaktionsendtemperatur bezeichnete Größe bevorzugt im Zulauf des Phasentrennapparates gemessen wird. Die Differenz zwischen Start- und Reaktionsendtemperatur (adiabate Temperaturdifferenz, folgend auch als $\Delta T_{adiabat}$ bezeichnet) hängt von der Art des nitrierten Kohlenwasserstoffes ab und von dem Mengenverhältnis, in dem die Mischsäure und der aromatische Kohlenwasserstoff eingesetzt werden. Ein niedriges Mengenverhältnis von Mischsäure und aromatischem Kohlenwasserstoff (sogenanntes Phasenverhältnis) ergibt eine hohe adiabate Temperaturdifferenz und hat den Vorteil, dass eine hohe Menge des aromatischen Kohlenwasserstoffs pro Zeiteinheit umgesetzt wird.

Unter ansonsten gleichen Bedingungen zeigt ein höherer Wert für $\Delta T_{adiabat}$ vollständigeren Umsatz an. Im erfindungsgemäßen Verfahren liegen die Werte für $\Delta T_{adiabat}$ bevorzugt zwischen 25 K und 60 K, besonders bevorzugt zwischen 30 K und 45 K. Trotz der hohen adiabaten Temperaturdifferenz werden im erfindungsgemäßen Verfahren sehr gute Selektivitäten erzielt, was nach dem Stand der Technik nicht zu erwarten war.

Die Reaktionsendtemperaturen im Reaktor liegen bevorzugt zwischen 120 °C und 160 °C, besonders bevorzugt zwischen 130 und 140°C.

Für die Ausführung des erfindungsgemäßen Verfahrens wird bevorzugt ein Rohrreaktor eingesetzt, in dem mehrere Dispergierelemente über die Länge des Rohrreaktors verteilt angeordnet sind, die eine intensive Durchmischung und Redispergierung von Benzol, Salpetersäure und Schwefelsäure und Wasser gewährleisten. Ein solcher Reaktor, sowie die Form einsetzbarer Dispergierelemente sind beispielsweise in EP 0 708 076 B1 (Figur 2) und EP 1 291 078 A2 (Figur 1) beschrieben.

Ganz besonders bevorzugt geeignet ist ein Aufbau für den Rohrreaktor, wie er in EP 1 291 078 A2 (Figur 1, Absätze [0012] - [0013]) beschrieben ist. In EP 1 291 078 A2 kommen 3 bis 11 Dispergierelemente aus Tantal zum Einsatz (entsprechend 4 bis 12 Kammern; siehe Absatz [0012]), die jeweils 0,5 bar bis 4 bar Druckverlust erzeugen und für einen Massenstrom von 1 t/h jeweils 10 bis 25 Öffnungen aufweisen. Bei den Öffnungen kann es sich um Schlitze, Löcher oder Bohrungen handeln. In dem vorliegenden erfindungsgemäßen Verfahren können diese Parameter ebenfalls realisiert werden, um eine Koaleszenz der Phasen zu vermeiden und den Durchmesser der organischen Tröpfchen in der Säurephase klein zu halten. In der EP 1 291 078 A2 sind die Dispergierelemente so ausgelegt, dass der mittlere Tröpfchendurchmesser kleiner als 200 μm, besonders bevorzugt kleiner als 120 μm ist und, wie der Fachmann aus den Beispielen berechnen kann, in dem Reaktor aus emailliertem Stahl ein Druck von bis zu 10 bar erreicht wird. In dem vorliegenden erfindungsgemäßen Verfahren sind bei Verwendung eines zu EP 1 291 078 A2 äquivalenten Reaktoraufbaus die Dispergierelement so auszulegen, dass der absolute Druck vor dem Reaktor mindestens 15 bar beträgt, sofern der absolute Druck in der Gasphase des Phasentrennapparts kleiner oder gleich 1,0 bar ist.

Bevorzugt weisen die Dispergierelemente in Strömungsrichtung der Reaktanden abnehmende Druckverluste auf. Besonders bevorzugt weisen das - in Strömungsrichtung der Reaktanden - zweite und jedes weitere Dispergierelement höchstens 80 % des Druckverlustes des jeweils vorangehenden Dispergierelementes auf.

Für die Ausführung des erfindungsgemäßen Verfahrens werden Reaktoren eingesetzt, deren zulässiger absoluter Betriebsdruck bevorzugt mindestens 18 bar, besonders bevorzugt mindestens 21 bar, beträgt. Diese Reaktoren können beispielsweise aus hochlegierten Stählen gefertigt sein. Die Beständigkeit geeigneter Edelstähle ist unter Nitrierbedingungen insbesondere dadurch gegeben, dass die zur Nitrierung eingesetzte Salpetersäure passivierend wirkt. Auch können diese Reaktoren aus emailliertem Stahl gefertigt sein. Eine Kombination von hochlegierten und emaillierten Stählen ist ebenfalls möglich.

Durch das Durchströmen der Dispergierelemente nimmt der Druck der Flüssigkeit innerhalb des Reaktors ab. Dadurch ergibt sich eine weitere Ausführungsform für den Aufbau des Reaktors: So kann der vordere Teil für einen hohen zulässigen Betriebsdruck ausgelegt werden und der Teil, in dem der Druck deutlich geringer ist, für einen niedrigeren zulässigen Betriebsdruck ausgelegt werden.

Da Stahlemaille im Vergleich zu hochlegierten Stählen ein kostengünstiger Werkstoff ist, ist die Verwendung von Stahlemaille-Rohrsegmenten für den kompletten Aufbau des Rohrreaktors bevorzugt, da diese Ausführungsform besonders dem Ziel gerecht wird zu niedrigen Investitionskosten zu kommen. Um in einem Reaktor aus emailliertem Stahl absolute Betriebsdrücke von mehr als 15 bar realisieren zu können, kann es erforderlich sein, besondere Anforderungen an die Güte der Fertigung der zu verwendenden emaillierten Rohrsegmente zu stellen. So ist beispielsweise auf die Rechtwinkligkeit zwischen Bördel und Rohr zu achten. Auch kann es erforderlich sein, besondere Anforderungen an das emaillierte Flanschblatt zu stellen, insbesondere Unebenheiten sorgfältig zu entfernen. Durch Schleifen oder Polieren des emaillierten Flanschblattes kann eine optimale Auflagefläche für die Dichtungen geschaffen werden. Zusätzlich kann durch eine geeignete Verschraubung der Flansche eine gleichmäßige Flächenpressung erzielt werden. Um die erforderliche Druckzulassung zu erhalten, sind auch geeignete Dichtungen auszuwählen, die bevorzugt den überwiegenden Teil des Flanschblattes bedecken und die gegebenenfalls mittels einer Bandage zwischen den Flanschblättern zentriert werden können. Auch kann auf Dichtungen zurückgegriffen werden, die auf den Flächen und den Außenkanten verstärkt wurden, beispielsweise durch Glasfasergewebe auf den Flächen oder Stützringen an den Außenkanten.

Nach Durchlaufen des Nitrierreaktors wird das rohe, flüssige Reaktionsprodukt einem Phasentrennapparat zugeführt. Als Phasentrennapparate können alle dem Fachmann geläufigen Ausführungsformen eingesetzt werden. Bevorzugt wird die Trennung in einem Schwerkraftabscheider durchgeführt. Die im Phasentrennapparat erhaltenen Flüssigphasen, Rohnitrobenzol und Absäure, werden bevorzugt wie folgt aufgearbeitet:

Die Absäure wird üblicherweise einem Entspannungsverdampfer (auch Blitzverdampfer) zugeführt, in dem bei der Entspannung der Absäure in einen Bereich reduzierten Drucks hinein Wasser verdampft und so die Absäure gekühlt und aufkonzentriert wird. Bei adiabater Fahrweise der Nitrierung von Benzol mit Mischsäure ergibt sich der Vorteil, dass die Reaktionswärme der exothermen Reaktion dazu genutzt wird, die Absäure so stark zu erhitzen, dass im Entspannungsverdampfer gleichzeitig wieder die Konzentration und die Temperatur eingestellt werden kann, die die Schwefelsäure vor der Zumischung von Salpetersäure und Benzol aufwies.

Das im Phasentrennapparat erhaltene Rohnitrobenzol weist als Verunreinigungen noch Schwefelsäure, Wasser, Benzol sowie Nitrophenole und Dinitrobenzol auf, die durch geeignete Aufarbeitungsverfahren wie z. B. Wasch- und Destillationsstufen abgetrennt werden. Eine mögliche Ausführungsform dieser Aufarbeitung ist in EP 1 816 117 A1 (Absatz [0006]) beschrieben. Es sind aber auch andere Ausführungsformen möglich.

Die im Phasentrennapparate gebildeten Gase werden bevorzugt einem Abgassystem zugeführt.

Durch das erfindungsgemäße Verfahren wird eine Raum-Zeit-Ausbeute von bevorzugt mehr als 7,0 t Nitrobenzol pro Kubikmeter Volumen des Reaktionsraumes und Stunden *bei sehr geringen Nebenproduktgehalten* erreichbar. Der geringe Gehalt an Nebenprodukten trotz - infolge der hohen Raum-Zeit-Ausbeuten - großer adiabater Temperaturdifferenzen wird ermöglicht durch die Anwendung eines hohen Druckes vor dem Reaktor, der um 15 bar bis 25 bar über dem Druck in der Gasphase des Phasentrennapparates liegt.

Das erfindungsgemäße Verfahren wurde vorstehend am Beispiel von Nitrobenzol beschrieben. Der Fachmann kann die Erfindung jedoch auch leicht auf die Herstellung anderer Nitroaromaten ausweiten; z. B. auf die Herstellung von Dinitrotoluol durch Nitrierung von Toluol.

**Beispiele**

**Beispiel 1 bis 3 (nicht erfindungsgemäß), Beispiel 4 (erfindungsgemäß)**

Die nachfolgenden Beispiele wurden unter folgenden Bedingungen durchgeführt:

Alle Versuche wurden in einem Rohrreaktor mit einem Innenvolumen von 455 mL aus emailliertem Stahl durchgeführt. Der Rohrreaktor wurde vertikal montiert und von unten angeströmt. Der Rohrreaktor wies 10 Dispergierelemente aus Tantal auf, die nach oben hin abnehmende Druckverluste aufwiesen. Vor dem Rohrreaktor wurde mit einem Membranmanometer der herrschende Druck in der Mischsäure gemessen (pla), bevor Benzol mittels einer druckverlustarmen Lanze in die Mischsäure eingeleitet und der sich ergebende Strom in den Reaktor eingeführt wurde. p1a kann bei dem vorliegenden Versuchsaufbau mit dem Druck p1 des durch Kontaktierung von Benzol und Mischsäure erhaltenen Verfahrensproduktes bei Eintritt in den Reaktor gleichgesetzt werden. Die Schwefelsäure hatte stets eine Konzentration von 71 Massen-% und die Salpetersäure hatte stets eine Konzentration von 69,2 Massen-%. Die Mischsäure hatte stets eine Temperatur von 96 °C und das Benzol wurde vorgeheizt und hatte stets eine Temperatur von 80 °C. Die Reinheit des Benzols betrug stets mehr als 99 Massen-%. Nach Durchlaufen des Reaktors wurde das rohe Reaktionsprodukt in einen drucklos betriebenen statischen Phasentrennapparat eingeleitet.

Die in der Tabelle 1 aufgeführten Mengen an Benzol wurden mit der Mischung der angegebenen Mengen an Salpetersäure und Schwefelsäure umgesetzt. Der Benzolüberschuss betrug, bezogen auf das Produkt Nitrobenzol jeweils 6 Massen-%.

Am Ende des Rohrreaktors wurde die adiabate Endtemperatur gemessen und mittels der Mischtemperatur der Mischsäure- und Benzolströme die adiabate Temperaturdifferenz $\Delta T_{adiabat}$ berechnet. Das von Säure im Phasentrennapparat abgetrennte Nitrobenzol wurde auf seinen Gehalt an Dinitrobenzol und Nitrophenolen hin analysiert. Aus der Gruppe der Nitrophenole kann Pikrinsäure getrennt ausgewiesen werden. In allen Versuchen wurde die eingesetzte Salpetersäure vollständig (> 99,99 % laut ionenchromatographischer Analyse der Absäure auf Nitrat) umgesetzt. Im erfindungsgemäßen Beispiel 4 konnte mit einer Druckdifferenz von 15,8 bar eine Raum-Zeit-Ausbeute von mehr als 7,0 $t_{Nitrobenzol}/(m^3_{Reaktionsraum} \cdot h)$ und trotz hoher adiabater Temperaturdifferenz die geringsten Gehalte an Nebenprodukten realisiert werden!

Tabelle 1: Parameter und Resultate der Beispiele 1 bis 4.

| Beispiel: | 1 | 2 | 3 | 4 |
|---|---|---|---|---|
| Raum-Zeit-Ausbeute [$t_{Nitrobenzol}/(m^3_{Reaktionsraum} \cdot h)$] | 5,5 | 6,2 | 6,4 | 7,2 |
| HN0$_3$-Strom [g/h] | 1864 | 2089 | 2169 | 2410 |
| H$_2$S0$_4$-Strom [g/h] | 30000 | 34000 | 34000 | 38000 |
| Benzol-Strom [g/h] | 1760 | 1970 | 2050 | 2275 |
| Absoluter Druck bei Eintritt in den Reaktor (p1) [bar] | 10,7 | 13,4 | 13, 6 | 16, 8 |
| Absoluter Druck in der Gasphase des Phasentrenn-apparates (p2) [bar] | 1,0 | 1,0 | 1,0 | 1,0 |
| **Druckdifferenz p1 - p2 [bar]** | **9,7** | **12,4** | **12,6** | **15,8** |
| $\Delta T_{adiabat}$ [K] | 42,3 | 41,7 | 44,4 | 44,1 |
| Salpetersäureumsatz [%] | > 99,99 | > 99,99 | > 99,99 | > 99,99 |
| Gehalt an Dinitrobenzol [Massen-ppm] | 293 | 211 | 213 | 176 |
| Gehalt an Nitrophenolen (Summe) [Massen-ppm] | 2153 | 2169 | 2055 | 1973 |
| Gehalt an Pikrinsäure [Mas-sen-ppm] | 171 | 136 | 122 | 89 |

**Patentansprüche**

1.  Verfahren zur Herstellung von Nitrobenzol durch adiabate Nitrierung von Benzol mit Mischsäure enthaltend Schwefelsäure und Salpetersäure, wobei

    (i) Benzol und Mischsäure entweder

       a) getrennt voneinander
       oder
       b) gemeinsam, nachdem beide miteinander kontaktiert wurden
       in einen Reaktor eingeleitet werden, wobei im Fall a) mindestens einer der Reaktanden Benzol oder Mischsäure, im Fall b) das nach Kontaktierung von Benzol und Mischsäure erhaltene Verfahrensprodukt bei Eintritt in den Reaktor unter einem Druck p 1 steht;

       (ii) Benzol und Mischsäure im Reaktor in 1 bis 30 hintereinander angeordneten Dispergierelementen ineinander dispergiert werden;
       (iii) das Reaktionsprodukt nach Verlassen des Reaktors einer Phasentrennung in einem Phasentrennapparat, in dessen Gasphase der Druck p2 herrscht, unterworfen wird;
       **dadurch gekennzeichnet, dass**
       (iv) die Druckdifferenz p1 - p2 15 bar bis 25 bar beträgt.

2.  Verfahren nach Anspruch 1, wobei die adiabate Temperaturdifferenz zwischen der Temperatur im Reaktor nach mindestens 99%igem Umsatz an Salpetersäure und der Temperatur der vermischten Reaktanden Benzol und Mischsäure vor Einsetzen der Reaktion zwischen 25 K und 60 K liegt.

3.  Verfahren nach Anspruch 1, wobei 2 bis 20 Dispergierelemente eingesetzt werden.

4.  Verfahren nach Anspruch 3, wobei jedes Dispergierelement einen Druckverlust aufweist und die Druckverluste der Dispergierelemente in Strömungsrichtung der Reaktanden abnehmen.

5.  Verfahren nach Anspruch 4, wobei das in Strömungsrichtung der Reaktanden zweite und jedes weitere Dispergierelement höchstens 80 % des Druckverlustes des jeweils vorangehenden Dispergierelementes aufweisen.

**Claims**

1.  A process for the production of nitrobenzene by adiabatic nitration of benzene with mixed acid comprising sulfuric acid and nitric acid, the process comprising:

    (i) introducing benzene and mixed acid into a reactor either

       a) separately
       or
       b) together, after being brought into contact with one another, with at least one of the benzene and mixed acid reactants in case a), and the process product obtained after benzene and mixed acid have been brought into contact in case b), under a pressure p1 on entry into the reactor;

       (ii) dispersing the benzene and the mixed acid in one another in the reactor in from 1 to 30 dispersing elements arranged one behind the other;
       (iii) subjecting the reaction product, after it has left the reactor, to a phase separation in a phase separation apparatus, in which a gas phase pressure p2 prevails;
       in which
       (iv) the pressure difference p1 - p2 = from 15 bar to 25 bar.

2.  The process of Claim 1 in which the adiabatic temperature difference between the temperature in the reactor after at least 99% conversion of nitric acid and the temperature of the mixed benzene and mixed acid reactants before onset of the reaction is from 25 K to 60 K.

3. The process of Claim 1 in which from 2 to 20 dispersing elements are used.

4. The process of Claim 3 in which each dispersing element exhibits a pressure drop, and the pressure drops of the dispersing elements decrease in the direction of flow of the reactants.

5. The process of Claim 4 in which the second and each subsequent dispersing element in the direction of flow of the reactants exhibit at most 80% of the pressure drop of the respective preceding dispersing element.


**Revendications**

1. Procédé pour la production de nitrobenzène par nitration adiabatique de benzène avec de l'acide lactique contenant de l'acide sulfurique et de l'acide nitrique, dans lequel

   (i) on introduit dans un réacteur du benzène et de l'acide lactique soit

       a) séparément l'un de l'autre
       soit
       b) ensemble, après les avoir mis en contact l'un avec l'autre dans le cas a) au moins l'un des partenaires réactionnels benzène ou acide lactique, dans le cas b) le produit de processus obtenu après mise en contact du benzène et de l'acide lactique se trouvant sous une pression p1 lors de l'entrée dans le réacteur ;

   (ii) dans le réacteur le benzène et l'acide lactique sont dispersés l'un dans l'autre dans 1 à 30 éléments de dispersion placés les uns derrière les autres,
   (iii) le produit de réaction, après avoir quitté le réacteur, est soumis à une séparation de phases dans un appareil de séparation de phases, dans la phase gazeuse duquel règne la pression p2 ;
   **caractérisé en ce que**
   (iv) la différence de pression p1 - p2 vaut de 15 bars à 25 bars.

2. Procédé selon la revendication 1, dans lequel la différence de température adiabatique entre la température dans le réacteur après une conversion d'au moins 99 % de l'acide nitrique et la température des partenaires réactionnels benzène et acide lactique mélangés, avant le début de la réaction, est comprise entre 25 K et 60 K.

3. Procédé selon la revendication 1, dans lequel on utilise 2 à 20 éléments de dispersion.

4. Procédé selon la revendication 3, dans lequel chaque élément de dispersion présente une perte de pression et les pertes de pression des éléments de dispersion diminuent dans le sens d'écoulement des partenaires réactionnels.

5. Procédé selon la revendication 4, dans lequel le deuxième élément de dispersion et chaque élément de dispersion suivant dans le sens d'écoulement des partenaires réactionnels présentent au maximum 80 % de la perte de pression de l'élément de dispersion précédent respectif.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2256999 A
- EP 0436443 B1
- EP 0771783 B1
- US 6562247 B2
- EP 0373966 B1
- EP 0489211 B1
- EP 0779270 B1

- EP 1291078 A1
- EP 1291078 A2
- EP 2070907 A1
- US 4091042 A
- EP 0708076 B1
- EP 1816117 A1